# EUROPEAN PATENT APPLICATION

(11) **EP 3 006 035 A1**
(43) Date of publication of application: **13.04.2016**
(21) Application number: 15189088.6
(22) Date of filing: 09.10.2015
(51) Int. Cl.: A61K 36/80, A61K 36/53, A61P 29/00, A61P 31/00

(54) **CICATRIZING COMPOSITION FOR THE TREATMENT AND RESOLUTION OF EXTERNAL LESIONS**

(30) Priority: 09.10.2014 IT ME20140002
(71) Applicant: Foti, Salvatore, 98054 Furnari (IT)
(72) Inventor: Foti, Salvatore, 98054 Furnari (IT)
(74) Representative: Coppo, Alessandro

(57) **Abstract**

The present invention concerns a composition for the treatment of skin lesions of a human being or an animal comprising a synergic association of a vegetable extract of the plant Verbascum and a vegetable extract of the plant Calamintha nepeta in a physiologically acceptable carrier or solvent. The composition of the invention has an oleolite-based liquid form or a beeswax-based solid form.

## Description

### FIELD OF THE INVENTION

The present invention originates in the field of products for dermatological use.

Specifically, the invention originates in the field of products for medical use for the treatment and repair of external lesions, in particular lesions with an exudative component which is typical of inflammation.

The composition of the invention finds application in human and veterinary applications.

Typically, the composition of the invention has cicatrizing, antiseptic, anti-inflammatory, antibacterial and antalgic properties and repellent properties against myasigenic and non-myasigenic diptera.

### BACKGROUND OF THE INVENTION

Nowadays, both in human and veterinary medicine, common disinfection of external lesions is performed according to a protocol that entails removal of the ischemic, necrotic, degenerative or suppurative soft parts followed by disinfection with disinfectants based on disinfecting preparations containing iodine or benzalkyl-chlorides or hydrogen peroxide-based compounds which, however, are all variously damaging to the skin, although considered essential for removing and killing the bacterial components that infest the lesions.

In certain cases a local and/or general antibiotic treatment is performed with powder, ointment, liquid or spray products, partially damaging to the skin and not conducive to cicatrization, in order to prevent and/or combat the development of bacterial infections.

Lastly, to maintain the lesions and avoid hardening and dehydration of the eschars, especially in very extensive lesions or lesions caused by burns, gauzes soaked in fatty humectants with cicatrizing activity are applied, but these are extremely costly.

During the maintenance therapy, the processes described above are applied as required while awaiting activation of the spontaneous repair processes.

In particular, the secretions and exudations - more or less abundant according to the degree and nature of the lesion - and any suppurated or necrotic soft parts are removed daily with disinfection and application of the products listed above.

In the veterinary ambit, the wounds of animals are often exposed to attacks by myasigenic diptera and non-myasigenic flies which contribute to importing infections onto the wound, even simply by alighting on the lesions.

A zootechnical practice which is widespread but not very effective in addition to being dangerous, is to sprinkle animal lesions with creolin which performs the function of killing the larvae and keeping away the flies for a brief period of time, however it necrotizes and destroys the tissues undergoing cicatrization.

Another remedy in the zootechnical sector is the use of various insecticides, directly on the larvae-infested wounds; the absorption of these compounds on the surface of the open lesions, often if extensive and slow to heal, is very often considerable and can involve risks of both acute and chronic intoxication in the animals.

Furthermore, the use of insecticides also exposes the operators to intoxications, in addition to increasing the pollution load of the environment and the agri-food supply chain when the animals treated are directly or indirectly intended for food production.

So far no medical-surgical products are known, used in official medicine, that manifest recognised and proven cicatrizing, anti-inflammatory, antiedema, antalgic and repellent properties in veterinary use.

At the same time, the treatment of extensive lesions, in particular during the periods in which flies are active, is difficult to manage in small or large animals out to pasture.

Also in humans the treatment of external lesions represents a serious problem especially in developing countries where the hygiene and environmental conditions are totally inadequate and the risk of exposure to parasitic and/or bacterial complications is very high and very frequent, particularly in weak and debilitated persons.

### SUMMARY OF THE INVENTION

One of the objects of the invention is to provide a composition useful in the treatment and/or cicatrization of external lesions, the active ingredients thereof are of vegetable origin and therefore substantially devoid of side effects.

A further object of the invention consists in providing a composition for external use for the treatment of skin lesions outside the hospital environment without the risk of generating secondary reactions.

These objects are met by providing a composition for use in the treatment of a skin lesion comprising the combination of an extract of the plant Verbascum and an extract of the plant Calamintha.

The present invention originates from the finding that a combination of an extract of the plant Verbascum with an extract of the plant Calamintha provides a surprising curative action for skin lesions, together with a synergistic cicatrizing, antibacterial and antiedema action.

The cicatrizing action of the composition of the invention includes the triggering, strengthening and acceleration of the physiological skin regeneration processes.

In this application, by the term "synergistic or synergism" it is meant an activity which is greater than the sum of the activities of the individual active ingredient. In particular, synergism occurs when at least two substances or active ingredients interact in a way that strengthens or increases one or more of their effects. Therefore, two substances or active ingredients that produce evidently similar effects will sometimes produce exaggerated or reduced effects when used simultaneously, and a quantitative evaluation is necessary to distinguish these cases from a simple additive action.

The applicants have furthermore observed that the composition based on extracts of the two plants according to the invention also has anti-inflammatory, antibiotic, analgesic, antiedema and repellent properties, and is particularly active against myasigenic and non-myasigenic diptera. The application of the composition of the invention on skin lesions, of any type and extent, accelerates and strengthens the physiological healing process, making a simultaneous local antibiotic treatment superfluous. Typically, the cicatrizing action of the composition of the invention is accompanied by a repellent effect against myasigenic diptera.

Typically, the composition of the invention is useful in the treatment of external skin lesions, especially with an exudative component typical of inflammations.

The composition of the invention may be useful in the treatment of external lesions of any type, for example wounds, varicose veins, bruises, burns, haemorrhoids, thrombophlebitis, rheumatoid arthritis or in skin disorders where it is necessary to drain, stimulate the lymphatic circulation and promote the elimination of excess liquids, as in cellulite.

The composition of the invention acts on the elastic or endothelial component of the venous wall and reduces the permeability of the blood vessels, limiting the outflow of liquids.

According to another aspect, a composition according to the invention is provided for use in the treatment of thrombophlebitis, varicose veins, arthritis, bruises, burns, rheumatoid arthritis or cellulite and in general in disorders wherein it is necessary to drain, stimulate the lymphatic circulation and promote the elimination of excess liquids.

### BRIEF DESCRIPTION OF THE FIGURES

Further characteristics of the invention will become clear from the following detailed disclosure, with reference to the attached figures in which:

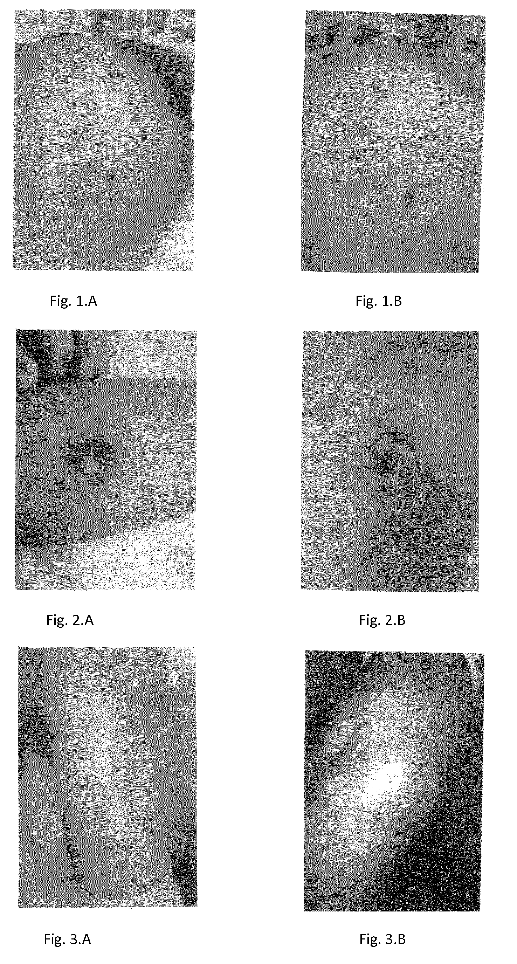

Fig. 1.A specifically shows the aspect of an infected lesion in the scalp of a patient.
Fig. 1.B shows the same patient after three days of treatment; only a slight reddening is apparent in the area where the lesions were previously present.
Fig. 2.A highlights a burn of second degree on the arm of a patient and (fig. 2.B) after two days the lesion appears well healed.
Fig. 3.A shows the elbow of a patient with purulent infection, healed after one week of treatment (fig. 3.B).
Fig. 4.A shows a *folliculitis barbae*, the manifestations of which have almost disappeared after three days of treatment (fig. 4.B).
Fig. 5.A shows a wasp sting in the shoulder of a patient. After only one day of treatment it appears well healed (fig. 5.B).
Fig. 6.A shows an extensive lacerated and contused lesion on the leg of a dog (Alsatian). After ten days of treatment it appears well healed (Fig. 6.B).

### DETAILED DISCLOSURE OF THE INVENTION

According to a first aspect, the present invention provides a composition for use in the treatment of a skin lesion comprising a synergic association of an extract of the plant Verbascum and an extract of Calamintha nepeta. It has been observed that the combination of the two extracts exerts surprising cicatrizing, anti-inflammatory, antiedema, repellent and biocidal properties.

One of the active components of the composition of the invention comprises a vegetable extract or a vegetable portion of Verbascum or a biologically active component contained in an extract of Verbascum, in particular rotenone, apigenin.

Typically, the plant extract or mixture of plant extracts of the invention comprises a fatty-based carrier, for example an oil like extra virgin olive oil in which the active components originating from the plant are present. Verbascum is a genus of plants belonging to the Scrophulariaceae family. Within the scope of the invention, by the term Verbascum it is meant any species belonging to the genus Verbascum.

For example, the genus Verbascum includes the species Verbascum Thapsus L., Verbascum nigrum, Verbascum blattaria.

the scope of the invention it is possible to use vegetable portions of Verbascum and/or an active component obtained from a species of Verbascum.

A suitable vegetable extract from a Verbascum plant is obtained by extracting or macerating a portion of Verbascum, typically the flowers, in a solvent oil. Typical solvent oils comprise vegetable oils such as olive oil, sunflower oil, maize oil, palm oil, sweet almond oil or grape seed oil.

By way of example, a suitable extract of Verbascum may be prepared by cutting up into small pieces a portion of plant, typically the flower, and placing it in contact with a vegetable oil, leaving it to macerate for example for a period ranging from 2 days to 2 months, typically 5 to 7 weeks. Typically, after this procedure the macerate obtained is then filtered and transferred to dark glass to avoid oxidisation processes caused by exposure to the natural light.

After a suitable maceration period, the macerated product is filtered, separating an extract into oil or oleolite rich in liposoluble substances extracted from the plant.

In certain embodiments the composition of the invention contains an extract of Verbascum or its biologically active components in a quantity ranging from 0.1 to 70% by weight, 0.5 to 60%, 2% to 40% by weight. Another component of the composition of the invention comprises a vegetable extract or a vegetable portion of Calamintha or a biologically active component contained in an extract of Calamintha.

Calamintha or Calamintha nepeta is a plant belonging to the genus Clinopodium. Within the scope of the invention, by the term Calamintha it is also meant Clinopodium nepeta.

Within the scope of the invention it is possible to use vegetable portions of Calamintha and/or an active component obtained from Calamintha.

A suitable vegetable extract from a Calamintha plant is obtained by extracting or macerating a portion of Calamintha in an oil-based solvent. Typical solvent oils comprise vegetable oils such as olive oil, sunflower oil, maize oil, palm oil, sweet almond oil or grape seed oil.

By way of an example a suitable extract of Calamintha can be prepared by cutting up into small pieces a portion of plant, typically leaves and flowers, and placing it in contact with a vegetable oil, leaving to macerate for example for a period ranging from 2 days to 8 weeks, typically 5 to 7 weeks.

After a suitable period of maceration, the macerated product is filtered, separating the oleolite of Calamintha rich in liposoluble substances extracted from the plant.

In certain embodiments, the composition of the invention contains an extract of Calamintha or its biologically active components in an amount ranging from 0.1 to 70% by weight, 0.5 to 60%, 2% to 40% by weight. The composition of the invention comprises a combination of substances of vegetable origin making available to the organism molecules compatible with the biological structures, to interact in an integrated manner with the tissues of the human body.

The substances or active ingredients contained in the extracts of the invention can be obtained with conventional extraction methods, for example extraction with solvent, extraction with supercritical fluids, distillation, enfleurage or percolation.

The applicants have furthermore observed that the combination of extracts from the plants Verbascum and Calamintha contain biologically active substances which act on the elastic component of the venous wall. The external application of the composition on the skin restores the functionality of the veins treated, increasing elasticity and tone.

In particular the active components of the composition of the invention also act on the endothelial component of the blood vessel where they are able to correct the permeability, limiting the outflow of liquids.

When the venous wall loses elasticity, dilating due to the increased blood pressure, the valve flaps are no longer able to close tightly and contain the reflux of blood downwards. The blood stagnates in the slack areas of the vessels, thus further increasing the pressure on the venous walls, progressively worsening the situation; this creates hyperalgesia, a vicious circle which gradually makes the valve defect irreversible, determining venous insufficiency.

The application of an effective amount of the composition of the invention in the areas of the skin where the veins are visible provides a treatment of the venous pathologies or disorders in which the vessels have an elasticity reduced to physiological level.

In some embodiments, the composition of the invention comprises further substances or active ingredients.

The invention further concerns compositions in which Azadirachtin A, a triterpenoid in the class of the natural limonoids, extracted from the ripe seeds of the Neem tree (Azadirachta indica), classified in class IV by the USA EPA and by the EEA, is added to the mixture of oleolites of Verbascum and Calamintha.

It has been observed that the incorporation of Azadirachtin A into the composition increases the biocidal and disinfecting properties of the composition of the invention.

According to certain embodiments, the Azadirachtin A is incorporated in the formulation of the invention in a concentration ranging from 100 ppm to 1000 ppm and oils q.s. for 1000 g. It has been observed that the presence of Azadirachtin A increases the insecticidal, acaricidal and nematicidal properties, and inhibits insect growth by acting as an antifeedant.

The toxicity of this additional compound is very low, to the extent that this compound is considered harmless to humans and vertebrates, including fish. The oral LD50 ascertained in rats is higher than 5000 mg/kg. According to some embodiments, the composition of the invention further comprises bee honey as a strengthener of the cicatrizing activity.

The compositions of the present invention comprise any composition produced by administering the association of active ingredients of the present invention and a physiologically or pharmaceutically acceptable carrier.

In some embodiments the composition of the invention comprises a physiologically and/or cosmetically acceptable carrier, diluent or excipient. Typically, the physiologically acceptable carrier of the composition of the invention is an excipient, carrier or diluent suitable for topical application. In the ambit of the present invention the term "carrier" refers to an excipient, carrier, diluent or adjuvant which can be present in the composition of the invention.

The term "physiologically acceptable" as used here means that the composition or its active components are suitable for cutaneous application in general and when applied do not result in undesired toxicity, allergic response, reddening, incompatibility, instability and similar reactions.

Within the scope of the invention the term oleolite means a solution or suspension in an oily carrier of the phytocomplexes present in the plants Verbascum and Calamintha. Typically the oily carrier is an oil of vegetable origin, preferably olive oil, even more preferably extra virgin olive oil.

A suitable physiologically acceptable carrier is olive oil with content of oleic acid (C18:1) 63-83%, palmitic acid (C16:0) 7-17%, linoleic acid (C18:2) < 13.5%, stearic acid (C18:0) 1.5-4%, palmitoleic acid (C16:1) 0.3-3%, linoleic acid (C18:3) ≤ 0.9%.

In some embodiments, the components or active ingredients contained in the composition of the present invention can be combined or mixed as active ingredients in an intimate mixture with a carrier or excipient according to the techniques of the cosmetics industry.

Typically the composition of the invention uses active components of natural or vegetable origin, substantially without side effects, when applied locally.

Typically, the composition of the invention can be in any form suitable for external or topical application.

In some embodiments, the composition for local administration is in a liquid or fluid form, for example in the form of a suspension, emulsion, typically oleolite.

The composition for topical application can also be in a solid, semisolid, fluid, or semifluid form at ambient temperature.

Suitable formulations in solid form include creams, salves, pastes, ointments, masks, transdermal patches and soap. Preferably the composition or preparation of the invention is in the form of oleolite, cream or soap. The soap may be produced by means of a cold process. According to some embodiments the carrier of the composition of the invention comprises fats solid at ambient temperature. In some embodiments said solid fats comprise beeswax.

For example the composition can be formulated in solid form by means of the addition of beeswax, as a carrier, for example in a quantity ranging from 7% to 15% by weight.

The cicatrizing therapeutic activity of the composition of the invention was clinically tested on individuals affected by ulcers and skin lesions.

In the clinical experimentation carried out, the composition subject of the invention was applied daily on external lesions as a substitute for other medical surgical products currently known in the state of the art.

After 24 hours from application, stimulation of the physiological granulation had occurred with early revascularization of the lesioned tissues, elimination of the ischemic and/or necrotic tissues, halting of the serous exudation (called white haemorrhage in the case of very extensive burns), reabsorption of the extravasation and halting of the bacterial-putrefactive proliferation. 24 hours after the first treatment, the lesions had the typical appearance of the activated repair process, with the lesioned surface covered by soft, elastic and adequately moist granulation tissue, and reduction, until complete cessation, of the serous exudation.

Furthermore the lesions treated were not subject to chapping and cracking due to dryness and dehydration of the exudate that forms on the surface being repaired; in fact the surface eschar, which forms following the exudation, does not become thick and hard as in lesions that heal spontaneously, but is an uninterrupted soft elastic surface layer which performs the vicarious function of the epidermis, protecting it from bacterial invasion so that it does not become infected.

Lastly, the daily medications are facilitated due to the marked soothing and pain-relieving properties of the composition.

The experimentation performed showed that it is possible to solve external lesions of any type and extent by acceleration and strengthening of the cicatricial process.

Application on the skin of the composition of the invention furthermore has a repellent effect on the myasigenic diptera that colonize the external wounds and lesions of animals and humans.

According to another aspect, a composition is provided as defined in any one of the embodiments previously described for veterinary use in preventing or treating infestations of myasigenic diptera which colonize the external wounds and lesions of animals.

### EXAMPLE 1

Formulation in liquid form of oleolite having the following formulation:

| | |
|---|---|
| Verbascum | 90% macerated in extra virgin olive oil (oleolite) |
| Calamintha nepeta | 10% macerated in extra virgin olive oil (oleolite) |

### EXAMPLE 2

Formulation in solid waxy form having the following formulation:

| | |
|---|---|
| Verbascum | 80% |
| Calamintha nepeta | 8% |
| Beeswax | 12% |

### EXAMPLE 3

The composition in the form of oleolite of example 1 was clinically

### tested on some individuals affected by skin ulceration.

The treatment entailed the application of a quantity adapted to bathe the lesioned skin part twice a day for a period varying from 5 to 10 days.

The results are highlighted in the attached figures from which the following can be observed.

Figures 1.A and 1.B show the scalp of an individual in which macerated areas with purulent exudation, with dimensions of 1 x 0.7 cm (Fig. 1.A), can be seen. Already after the first day, a decrease in the exudation is noted, with reduction of the edema surrounding the infected patch. On the third day a slight skin reddening is noted where the lesions were previously present (Fig. 1.B).

Figures 2.A and 2.B highlight a second degree burn, which occurred one week prior to our intervention, with central lesion having jagged macerated edges of approximately 1 cm by 1 cm, with coexistence on the edges of hyperchromic skin with areas of micro necrosis (Fig. 2.A). After two days of intervention with one single application per day, the necrotic area was replaced by new epithelialization, while the central area was replaced by granulation tissue (Fig. 2.B).

Figures 3.A and 3.B show a purulent lesion on the elbow of an individual affected by *locus minoris resistentiae* from which pus is discharged and the surrounding skin is reddened and hot (Fig. 3.A). Fig. 3.B shows the same lesion perfectly healed after a week of treatment.

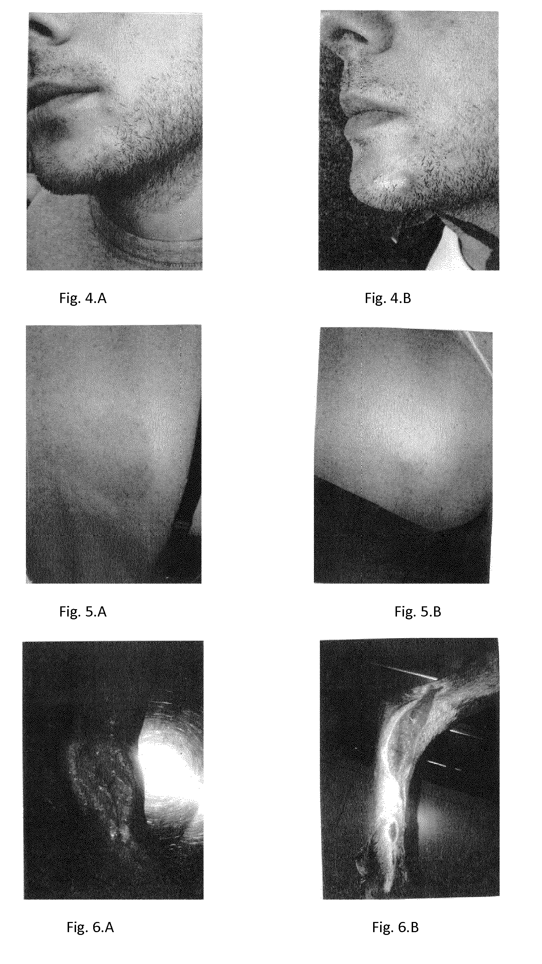

Figures 4.A and 4.B show a *folliculitis barbae*, which almost disappeared after three days of treatment (Fig. 4.B).

Figure 5.A shows a lesion on the shoulder resulting from a wasp sting with round, red, very itchy haematous lesion with dimensions of 1.5 x 2 cm.

After one day of treatment, the itchiness disappeared and only a slight hyperaemia of the area remained.

Fig. 6.A shows an extensive lacerated and contused lesion on the left leg of an Alsatian dog. After 10 days of treatment it appears perfectly healed. (Fig. 6.B).

The images of the attached figures show that the cicatrization times are limited and reduced to the minimum physiological times typical of the species due to the evident stimulation of the cicatricial processes and reduction of the complications that constantly accompany the lesions.

### EXAMPLE 4

### Veterinary use

A lacerated and contused lesion on the leg of a dog underwent the following treatment.

After surgical curettage of the lesioned area having a lozenge shape and size of approximately 7 cm, a layer (approx. 1-2 mm) of conventional fatty-based cream was applied containing the following active ingredients: Verbascum, Calamintha nepeta and Azadirachtin A in a concentration of approximately 800 ppm. The application was renewed every 12 hours until the wound had completely healed. A cicatrizing and anti-inflammatory effect on the lesioned part approximately 30% greater than the cicatrizing products commonly used for veterinary applications was observed. Furthermore the application of the preparation showed an insect-repelling effect which, during the local treatment, prevented the wound coming into contact with insects which are possible carriers of infection.

### EXAMPLE 5

### Treatment of sore with phlebopathy. Lesion site: right malleolus.

A bedsore with phlebopathy having dimensions of 3 x 0.7 cm with exudate and granulation tissue was treated with a cream based on fatty material containing Calaminth 10% Verbascum 75%, beeswax 15%. The treatment entailed the local application of a layer of approx. 1-2 mm twice a day for a period of 7 days.

At the end of the treatment period, almost complete healing and remission of the pain symptoms had occurred.

### EXAMPLE 6

### Treatment of plaque psoriasis

Plaque psoriasis on the elbow of a patient having typical whitish colouring with jagged red edges and dimensions of approx. 15 cm x 10 cm was treated with soap based on Verbascum 70% Calamintha nepeta 10% in a cold saponification process.

The treatment entailed application of the product on the lesion once a day. After the first day of treatment, the colour of the plaque changed from the typical whitish colouring to red. During the following days the skin colouring changed from red to the typical fleshy pink colour which blended in with the colour of the surrounding skin areas.

## Claims

1. Composition for topical use in the treatment of a skin lesion of a human being or an animal comprising a synergic combination of a vegetable plant extract of Verbascum and a vegetable plant extract of Calamintha nepeta and a physiologically acceptable carrier or solvent.

2. Composition for use according to claim 1 as a cicatrizant and or antiedema and or anti-inflammatory agent of a skin lesion.

3. Composition for use according to claim 1 or 2 wherein the physiologically acceptable solvent is a vegetable oil, preferably olive oil.

4. Composition according to claim 3 in oleolite liquid form.

5. Composition for use according to claim 1 or 2 in solid or semi-solid form.

6. Composition according to claim 5 comprising beeswax as a physiologically acceptable carrier.

7. Composition for use according to any one of the claims 1-4 **characterised in that** it comprises a vegetable extract of the plant Verbascum in an amount ranging from 2 to 40% by weight and a vegetable extract of the plant Calamintha nepeta in an amount ranging from 2 to 40% by weight.

8. Composition according to any one of the claims 1-7 for veterinary use as a cicatrizant of skin lesions and diptera repellent in animals.

9. Composition for use according to claim 8 further comprising Azadirachtin A.

10. Composition according to any one of the claims 1-9 in the form of a medical product.
